(19) Europäisches Patentamt
European Patent Office
Office européen des brevets

(11) Publication number: 0 008 813
B1

(12) **EUROPEAN PATENT SPECIFICATION**

(45) Date of publication of patent specification: 01.12.82

(51) Int. Cl.³: C 07 D 209/52

(21) Application number: 79200348.5

(22) Date of filing: 27.06.79

# (54) Processes for the preparation of 3-azabicyclo(3.1.0)hexane derivatives.

(30) Priority: 06.07.78 US 922408

(43) Date of publication of application:
19.03.80 Bulletin 80/6

(45) Publication of the grant of the patent:
01.12.82 Bulletin 82/48

(84) Designated Contracting States:
BE CH DE FR GB IT NL SE

(56) References cited:
DD - A - 133 943
DE - A - 2 646 188
DE - A - 2 653 251

(73) Proprietor: SHELL INTERNATIONALE RESEARCH MAATSCHAPPIJ B.V.
Carel van Bylandtlaan 30
NL-2596 HR Den Haag (NL)

(72) Inventor: Kollmeyer, Willy Dietrich
1008 Stratford Lane
Modesto, California 95350 (US)

(74) Representative: Rogers, Roy Charles et al,
4 York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 008 813 B1

# Processes for the preparation of 3-azabicyclo[3.1.0]hexane derivatives

This invention relates to processes for the preparation of certain 3-azabicyclo[3.1.0]hexane derivatives.

2-Carboxy-3-azabicyclo[3.1.0]hexane and certain of its salts and esters have very valuable biological properties, being capable of sterilising the male anthers of plants. These compounds are, however, extremely difficult to synthesize.

The Applicants have now found a convenient method synthesis starting from 3-azabicyclo[3.1.0]hex-2-ene.

The present invention provides a process for the preparation of 2-cyano-3-azabicyclo[3.1.0]hexane, which has the formula

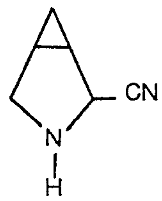

(I)

characterised in that it comprises reacting 3-azabicyclo[3.1.0]hex-2-ene and/or the trimer thereof with an alkali metal bisulphite to form its bisulphite adduct, and treating said adduct with an alkali metal cyanide. Preferably the alkali metal bisulphite is sodium bisulphite, and preferably the alkali metal cyanide is sodium cyanide. These salts are preferably used in aqueous solution.

Suitably an organic solution of 3-azabicyclo[3.1.0]hex-2-ene and/or trimer thereof is treated with an aqueous solution of sodium bisulphite, thus forming the 2-bisulphite adduct, at about room temperature. The resulting two-phase mixture may then be treated with an alkali metal cyanide, suitably in aqueous solution, at about room temperature, to form a solution of 2-cyano-3-azabicyclo[3.1.0]hexane in the organic phase. The product may be isolated by separating the two phases, removing the organic solvent and extracting the residue with a solvent, for example an ether, and removing the solvent from the extract. Preferably the reactants in both stages of the process are used in substantially equimolar amounts.

The 3-azabicyclo[3.1.0]hex-2-ene and/or its trimer may be prepared by dehydrohalogenation of 3-bromo- or 3-chloro-3-aza-bicyclo[3.1.0]hexane, using a suitable dehydrohalogenating agent. The product may if desired be isolated, or it may be used *in situ* for the subsequent process according to the invention, suitably after removal of inorganic salts from the reaction mixture.

The dehydrohalogenation may be carried out using any suitable dehydrohalogenating agent, for example a strong organic base such as triethylamine or pyridine in non-aqueous solution, or a strong inorganic base, for example sodium hydroxide, in aqueous or non-aqueous solution. Suitable polar solvents are for example ethers, alcohols, or water. Especially suitable reagents are alcoholic, preferably lower alkanolic, solutions of an alkali metal hydroxide or alkoxide. The reaction is preferably carried out at a temperature of up to 150°C, preferably at a temperature in the range of 0° to 80°C. The process can conveniently be carried out at reflux temperature of the solvent used. Lower temperatures, for example 0 to 20°C, may be convenient.

It is believed that the dehydrohalogenation process leads to a solution consisting largely of the monomer 3-azabicyclo[3.1.0]hex-2-ene, which has the formula

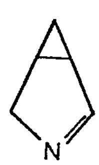

(II)

On removal of the solvent, a solid is produced which has a complex NMR spectrum and is believed to be the trimer:

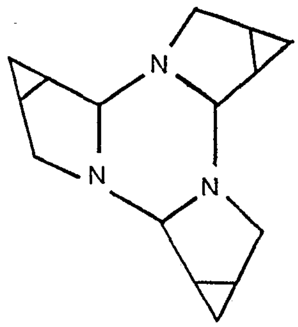

(IIa)

When the solid trimer is re-dissolved, a solution is formed in which the trimer is in equilibrium with the monomer, the concentration of each species being dependent on the dilution of the solution. The process according to the invention may be carried out irrespective of the relative proportions of monomer and trimer in the reaction solution.

3-Chloro- or 3-bromo- 3-azabicyclo[3.1.0]hexane may for example be prepared by the chlorination or bromination of 3-azabicyclo[3.1.0]hexane or an acid addition salt, for example the hydrochloride, thereof.

Any suitable chlorinating or brominating agent containing "active" or "positive" halogen may be used. This kind of halogenating agent is described and exemplified in detail in US patent 3,449,421. Common specific chlorinating agents of this type are N-chlorosuccinimide, N-chlorourea, and derivatives of hypochlorous acid, such as sodium hypochlorite, calcium hypochlorite, and tertiary-butyl hypochlorite. The chlorination or bromination can be conducted in a single solvent, such as an ether, or it may be done in a two (liquid) phase system such as a liquid hydrocarbon/water system. Suitably the reaction can be conducted at about, or somewhat below, room temperature. The product may be isolated from the crude reaction mixture by conventional techniques; however, where the treatment is carried out in an ether, the resulting solution of the product can be used in the next step of the process of the invention.

An acid addition salt for example the hydrochloride salt, can be chlorinated by treatment with an aqueous solution of an alkali metal hypochlorite, in the presence of a base, for example sodium bicarbonate, which serves to generate the free base *in situ*. Preferably, the hydrochloride salt is added to a mixture of the solution of the chlorinating agent and the bicarbonate, at a temperature of from about 0°C to about 15°C. The product may be isolated from the final reaction mixture by extraction with a solvent, such as an ether, the solution being suitable for use in the next step of the process of the invention.

Thus the invention includes a process for the preparation of 2-cyano-3-azabicyclo[3.1.0]hexane which comprises:

(i) reacting 3-azabicyclo[3.1.0]hexane or an acid addition salt thereof with a suitable chlorinating or brominating agent to produce 3-chloro- or 3-bromo- 3-azabicyclo[3.1.0]hexane;

(ii) dehydrohalogenating at least part of the resulting compound using a suitable dehydrohalogenating agent to produce 3-azabicyclo[3.1.0]hex-2-ene and/or the trimer thereof;

(iii) reacting at least part of the resulting compound with sodium bisulphite to form the bisulphite adduct; and

(iv) reacting at least part of the bisulphite adduct with an alkali metal cyanide to produce 2-cyano-3-azabicyclo[3.1.0]hexane.

In 2-cyano-3-azabicyclo[3.1.0]hexane, the 2-cyano group may be cis- or trans- to the bridge methylene group, and in addition, for each of these gemoetric isomers a pair of optical isomers exists due to the asymmetry of the 2-carbon atom. In some applications of the process according to the invention, certain isomers may be formed in preference to other isomers.

2-Cyano-3-azabicyclo[3.1.0]hexane may be hydrolyzed or alcoholized to form 2-carboxy-3-azabicyclo[3.1.0]hexane which has the formula

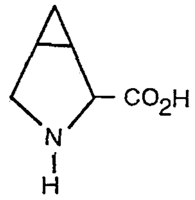

(III)

or a salt and/or ester thereof. Such compounds have interesting pollen-suppressing and plant growth regulating properties. The hydrolysis may for example be carried out by refluxing the cyano compound in the presence of an aqueous acid. This hydrolysis may lead to an acid addition salt of the free acid (III).

Alternatively, the cyano compound may be treated with barium hydroxide to form the barium salt of the acid III, which may in turn be treated with sulphuric acid to produce the free acid III. Refluxing of the cyano compound in the presence of an alcohol and dry hydrogen chloride produces in ester of the free acid III or the hydrochloride thereof.

3-Azabicyclo[3.1.0]hexane may be prepared by the method described in our co-pending application No. 79200347.7 (Publication No. 0007128), viz.

(i) reacting cis 1,2-cyclopropane dicarboxylic acid or the anhydride thereof with benzylamine to produce 3-benzyl-3-azabicyclo[3.1.0]hexan-2,4-dione;

(ii) reacting this compound with a complex aluminium hydride reducing agent thereby selectively reducing the carbonyl groups, to produce 3-benzyl-3-azabicyclo[3.1.0]hexane; and

(iii) converting this compound into 3-azabicyclo[3.1.0]hexane by hydrogenolysis of the benzyl bond.

Full details of this procedure are given in our co-pending application.

The following Examples illustrate the invention.

## Example 1

*(cis, trans) 2-cyano-3-azabicyclo[3.1.0]hexane* (1)

(A) A mixture of 11.8 g (0.9855 mol) of N-chlorosuccinimide, 4.16 g (0.05 mol) of 3-azabicyclo[3.1.0]hexane, prepared by the procedure of Example 1 of our co-pending application No. 79200347.7 (Publication No. 0007128), and 250 ml ether was stirred at room temperature for 2½ hours. After filtration, the ethereal solution was washed with water (2 × 100 ml) and brine (1 × 50 ml). The dried ($MgSO_4$) filtrate was carefully concentrated to about 40 ml total volume (40 cm Vigreux column). The remaining solution, containing 3-chloro-3-azabicyclo[3.1.0]hexane, was added dropwise (30 min) to a cooled (ice-bath) and stirred solution of 3.30 g (0.05 mol) of 85% potassium hydroxide in 25 ml of absolute ethanol. The white suspension was then stoppered and stirred at room temperature overnight (16 hours). Filtration removed inorganic salts, which were washed with a little ether. The combined filtrate and ether washings, which contained 3-azabicyclo[3.1.0]hex-2-ene, were treated with 5.20 g (0.05 mol) of sodium bisulphite in 25 ml water. After stirring vigorously for 1 hour at room temperature, the two-phase mixture containing the bisulphite adduct of 3-azabicyclo[3.1.0]hex-2-ene, was treated with 2.58 g (0.05 mol) of 95% sodium cyanide as a solid for 1 hour at room temperature. The upper organic layer was decanted and the aqueous layer was further extracted with ether (2 × 100 ml, decantation). The combined organic layer and ether extracts were concentrated on the rotary evaporator (water aspirator pressure, 70°C). The oily residue was diluted with 75 ml ether causing a small aqueous phase to separate. The resulting two-phase mixture was dried ($MgSO_4$) and the solvent was evaporated under reduced pressure to give *1*, as a light yellow oil. An analytical sample of 1 was a colourless oil, bp: 62°C (0.67 $N.m^{-2}$) (0.005 Torr.).

(B) 56.9 g (0.476 mol) of 3-azabicyclo[3.1.0]hexane hydrochloride prepared by the procedure of Example 1 of our copending application No. 79200347.7 (Publication No. 0007128), were added to 150 ml of a saturated solution of potassium hydroxide in water. The separated oil was dissolved in 200 ml of ether, the solution was dried ($MgSO_4$), and 112.5 g (0.842 mol) of N chlorosuccinimide in 800 ml of ether was added. The mixture was stirred for 3 hours at room temperature, then filtered, washed with water and sodium chloride solution, dried ($MgSO_4$) and carefully concentrated to about 200 ml volume in a Vigreux column. The resulting solution was added dropwise over a 30 minute period to a cooled (ice-bath) and stirred solution of 31.4 g (0.476 mol) of 85% potassium hydroxide in 200 ml of absolute ethanol. The resulting suspension was stirred at room temperature overnight, then filtered to remove inorganic salts. The filtrate was treated with 49.5 g (0.476 mol) of sodium bisulphite in 200 ml of water. After stirring for 75 minutes at room temperature, the two-phase mixture was treated with 24.6 g (0.476 mol) of 95% sodium cyanide. The mixture was stirred for 2 hours at room temperature, the organic phase was separated and the aqueous phase was extracted with ether. The combined organic phase and extract was concentrated under reduced pressure. The residue was diluted with 200 ml of ether. A small aqueous phase separated. The two-phase mixture was dried ($MgSO_4$) and stripped of solvent. The residue was distilled to give *1*, as a colourless liquid, bp: 58—61°C (1.33 $N.m^{-2}$) (0.01 Torr.).

(C) 12.6 g (0.15 mol) of sodium bicarbonate was added to a cooled (ice-bath) and stirred solution of 5.25% aqueous sodium hypochlorite, 212.7 g (0.15 mol). When most of the bicarbonate had dissolved, 12.0 g (0.10 mol) of 3-azabicyclo[3.1.0]hexane hydrochloride were added. The mixture was stirred (with cooling) for 1 hour and the resulting suspension was extracted with ether. The extract was dried ($MgSO_4$), and divided into two parts. One part was distilled in an attempt to isolate the 3-chloro-3-azabicyclo[3.1.0]hexane. However, the pot residue decomposed suddenly and vigorously when most of the ether had been distilled off, at 36°C and ambient pressure. The other part of the extract was concentrated to about 25 ml, and was added dropwise to a cooled (ice-bath) and stirred solution of 3.30 g (0.05 mol) of 85% potassium hydroxide in absolute ethanol. From this point on, the procedure followed that described in procedure B, above. *1* was obtained as a colourless oil, bp: 52—54°C (0.67 $N.m^{-2}$) (0.005 Torr.).

Example 2

*(± -cis) 2-carboxy-3-azabicyclo[3.1.0]hexane hydrochloride* (2) and the *(± -trans)-isomer* (3)

a mixture of 34.6 g (0.319 mol) of *1*, 102.8 g (0.325 mol) of barium hydroxide octahydrate, and 500 ml of water was refluxed for 7 hours. The mixture was cooled, and then was carefully neutralised to pH 6 with 33.2 g (0.325 mol) of 96% sulphuric acid in 500 ml of water. Celite (Registered Trademark) was added and the mixture was filtered. The solvent was evaporated and the residue was extracted with hot ethanol. The undissolved solid (2A) was an approximately 2/1 mixture of (± -trans)- and (± -cis)- 3-azabicyclo[3.1.0]hexane-2-carboxylic acid. The solid obtained from evaporation of the solvent from the extract (2B) was an approximately 2.2/1 mixture of the (± -cis)- and (± -trans)-isomers.

2B was subjected to chromatography on a cation exchange resin, using 1.5N hydrochloric acid as eluent, to give *2*, as a solid, mp. 226—228°C (with gas evolution), as the more mobile isomer. The less mobile isomer was *3*, mp 202—206°C (with gas evolution).

Example 3

*cis* and *trans 2-cyano-3-azabicyclo[3.1.0]hexane*

Two 10 litre stirred reaction flasks were blanketed with nitrogen and each charged with N-chlorosuccinimide (2.09 kg, 15.6 M) and diethyl ether (6.5 litres). 3-Azabicyclo[3.1.0]hexane, prepared as in Example 2 of our co-pending application No. 79200347.7 (Publication No. 0007148) (1.097 kg, 13.1 M) was added over one hour, the mildly exothermic reaction being controlled between 21—24°C by iced-water cooling. After addition was complete stirring was continued at ambient temperature for a further 3½ hours. Succinimide was filtered, each filter cake was washed twice with ether (1.25 litre portions), and the combined ethereal solutions, after washing in a stirred 50 litre vessel with demineralised water (4.5 litres) followed by saturated brine (2.3 litres), were used in the dehydrochlorination step.

A solution of sodium hydroxide (1.05 kg, 26.2 M) in methanol (6.5 litres) was placed in a 50 litre stirred reactor and a nitrogen blanket maintained. The ether solution of 3-chloro-3-azabicyclo[3.1.0]hexane was then added over a period of 1½ hours whilst maintaining the temperature between 18—28°C by iced water cooling. Reaction continued exothermically for a further 2 1/4 hours with the temperature in the above range. After stirring overnight, sodium chloride was removed by filtration.

This ethereal solution of 3-azabicyclo[3.1.0]hex-2-ene was returned to the 50 litre reactor. Addition of sodium metabisulphite (2.78 kg, of 90% 13.2 M) in demineralized water (5.5 litres) followed over 35 minutes, the temperature being held in the range 10—25°C by ice cooling. Coolant was removed and stirring continued at ambient temperature for 1 3/4 hours. Next, sodium cyanide (1.36 kg, of 98%, 27.2 M) in demineralized water (4 litres) was added over 30 minutes, ice cooling holding the temperature between 20—25°C. Cooling was again discontinued with stirring for another 1½ hours at ambient conditions.

The solution was filtered, and solvent and most of the entrained water were removed on a rotary evaporator at 40—50°C/6666 $N.m^{-2}$ 50 mm Hg yielding crude 2-cyano-3-azabicyclo[3.1.0]hexane (2.363 kg) with a cis/trans ratio of 25/75. Distillation yielded the pure product, boiling point 62—64°C at a pressure of 26.66 $N.m^{-2}$ (0.2 mm Hg), with a cis/trans ratio of 32/68. The yield was 70% based on starting material.

## Claims

1. A process for the preparation of 2-cyano-3-azabicyclo[3.1.0]hexane characterized in that it comprises reacting 3-azabicyclo[3.1.0]hex-2-ene and/or the trimer thereof with an alkali metal bisulphite to form its bisulphite adduct, and treating said adduct with an alkali metal cyanide.

2. A process as claimed in claim 1, characterized in that the reaction is carred out using an organic solution of 3-azabicyclo[3.1.0]hex-2-ene and/or trimer thereof and aqueous solutions of the alkali metal bisulphite and the alkali metal cyanide.

3. A process as claimed in either claim 1 or claim 2, characterized in that the 3-azabicyclo[3.1.0]hex-2-ene and/or its trimer has been prepared by dehydrohalogenation of 3-bromo- or 3-chloro-3-azabicyclo[3.1.0]hexane using a suitable dehydrohalogenating agent.

4. A process as claimed in claim 3, characterized in that the dehydrohalogenating agent is an alcoholic solution of an alkali metal hydroxide or alkoxide.

5. A process as claimed in either claim 3 or claim 4, characterized in that the 3-bromo- or 3-chloro- 3-azabicyclo[3.1.0]hexane has been prepared by bromination or chlorination of 3-azabicyclo[3.1.0]hexane or an acid addition salt thereof.

6. A process as claimed in claim 5, characterized in that chlorination is effected using N-chlorosuccinimide, N-chlorourea, sodium or calcium hypochlorite or tertiary butyl hypochlorite.

7. A process as claimed in either claim 5 or claim 6, characterized in that the 3-azabicyclo[3.1.0]hexane or acid addition salt thereof has been prepared by:

(i) reacting cis 1,2-cyclopropane dicarboxylic acid or the anhydride thereof with benzylamine to produce 3-benzyl-3-azabicyclo[3.1.0]hexan-2,4-dione;
(ii) reacting this compound with a complex aluminium hydride reducing agent, thereby selectively reducing the carbonyl groups to produce 3-benzyl-3-azabicyclo[3.1.0]hexane; and
(iii) converting this compound into 3-azabicyclo[3.1.0]hexane by hydrogenolysis of the benzyl bond.

8. A process for the preparation of 2-carboxy 3-azabicyclo[3.1.0]hexane or a salt and/or an ester thereof, characterized in that it comprises hydrolyzing or alcoholizing 2-cyano-3-azabicyclo[3.1.0]hexane which has been prepared by a process as claimed in any one of claims 1 to 7.

**Revendications**

1. Procédé pour la préparation de 2-cyano-3-azabicyclo(3.1.0)hexane, caractérisé en ce qu'on fait réagir du 3-azabicyclo(3.1.0)hex-2-ène et/ou son trimère avec un bisulfite de métal alcalin de façon à former son produit d'addition de bisulfite, et on traite ce produit d'addition par un cyanure de métal alcalin.

2. Procédé selon la revendication 1, caractérisé en ce qu'on conduit la réaction en utilisant une solution organique de 3-azabicyclo(3.1.0)hexène et/ou de son trimère et des solutions aqueuses du bisulfite de métal alcalin et du cyanure de métal alcalin.

3. Procédé selon la revendication 1 ou 2, caractérisé en ce que le 3-azabicyclo(3.1.0)hex-2-ène et/ou son trimère ont été préparés par déshalogénhydratation de 3-bromo- ou 3-chloro-3-azabicyclo(3.1.0)hexane en utilisant un agent de déshalogénhydratation approprié.

4. Procédé selon la revendication 3, caractérisé en ce que l'agent de déshalogénhydratation est une solution alcoolique d'un hydroxyde ou alcoolate de métal alcalin.

5. Procédé selon la revendication 3 ou 4, caractérisé en ce que le 3-bromo- ou 3-chloro- 3-azabicyclo(3.1.0)hexane a été préparé par bromation ou chloration de 3-azabicyclo(3.1.0)hexane ou d'un sel d'addition d'acide de ce composé.

6. Procédé selon la revendication 5, caractérisé en ce qu'on effectue la chloration en utilisant du N-chlorosuccinimide, de la N-chloro-urée, de l'hypochlorite de sodium ou de calcium ou de l'hypochlorite de tert-butyle.

7. Procédé selon la revendication 5 ou 6, caractérisé en ce que le 3-azabicyclo(3.1.0)hexane ou son sel d'addition d'acide a été préparé par

(1) réaction d'acide cis 1,2-cyclopropane dicarboxylique ou de l'anhydride de cet acide avec la benzylamine de façon à produire de la 3-benzyl-3-azabicyclo(3.1.0)hexan-2,4-dione;

(2) réaction de ce composé avec un agent réducteur hydrure complexe d'aluminium, de façon à réduire sélectivement les groupes carbonyle pour produire du 3-benzyl-3-azabicyclo(3.1.0)hexane; et

(3) transformation de ce composé en 3-azabicyclo(3.1.0)hexane par hydrogénolyse de la liaison benzyle.

8. Procédé pour la préparation de 2-carboxy-3-azabicyclo(3.1.0)hexane ou d'un sel et/ou d'un ester de ce composé, caractérisé en ce qu'il comprend l'hydrolyse ou l'alcoolyse de 2-cyano-3-azabicyclo(3.1.0)hexane qui a été préparé par un procédé selon l'une quelconque des revendications 1 à 7.

**Patentansprüche**

1. Verfahren zur Herstellung von 2-Cyano-3-azabicyclo[3.1.0]hexan, dadurch gekennzeichnet, daß man 3-Azabicyclo[3.1.0]hex-2-en und/oder das Trimer davon mit einem Alkalibisulfit unter Bildung des Bisulfit-Addukts umsetzt und dieses Addukt mit einem Alkalicyanid behandelt.

2. Verfahren nach Anspruch 1, dadurch gekennzeichnet, daß man die Reaktion unter Verwendung einer organischen Lösung von 3-Azabicyclo[3.1.0]hex-2-en und/oder dem Trimer davon und einer wäßrigen Lösung des Alkalibisulfits und des Alkalixyanids durchführt.

3. Verfahren nach Anspruch 1 oder 2, dadurch gekennzeichnet, daß das 3-Azabicyclo[3.1.0]hex-2-en und/oder sein Trimer hergestellt worden sind durch Dehydrohalogenierung vom 3-Brom- oder 3-Chlor- 3-azabicyclo[3.1.0]hexan unter Verwendung eines geeigneten Dehydrohalogenierungsmittels.

4. Verfahren nach Anspruch 3, dadurch gekennzeichnet, daß das Dehydrohalogenierungsmittel eine alkoholische Lösung eines Alkalihydroxids oder alkoxids ist.

5. Verfahren nach Anspruch 3 oder 4, dadurch gekennzeichnet, daß das 3-Brom- oder 3-Chlor-3-azabicyclo[3.1.0]hexan hergestellt worden ist durch Bromierung oder Chlorierung von 3-Azabicyclo[3.1.0]hexan oder einem Säureadditionssalz davon.

6. Verfahren nach Anspruch 5, dadurch gekennzeichnet, daß die Chlorierung durchgeführt wird unter Verwendung von N-chlorsuccinimid, N-Chlorharnstoff, Natrium- oder Calciumhypochlorit oder tert.-Butylhypochlorit.

7. Verfahren nach Anspruch 5 oder 6, dadurch gekennzeichnet, daß das 3-Azabicyclo[3.1.0]hexan oder ein Säureadditionssalz davon hergestellt worden ist durch

(i) Umsetzung von cis-1,2-Cyclopropan-dicarbonsäure oder deren Anhydrid mit Benzylamin unter Bildung von 3-Benzyl-azabicyclo[3.1.0]hexan-2,4-dion;

(ii) Umsetzung dieser Verbindung mit einem komplexen Aluminiumhydrid-Reduktionsmittel, wodurch selektiv die Carbonylgruppen reduziert werden unter Bildung von 3-Benzyl-3-azabicyclo[3.1.0]hexan; und

(iii) Umwandlung dieser Verbindung in 3-Azabicyclo[3.1.0]hexan durch Hydrogenolyse der Benzylbindung.

8. Verfahren zur Herstellung von 2-Carboxy-3-azabicyclo[3.1.0]hexan oder einem Salz und/oder einem Ester davon, dadurch gekennzeichnet, daß man 2-Cyano-3-azabicyclo[3.1.0]hexan, das hergestellt worden ist nach einem der Ansprüche 1 bis 7, hydrolysiert oder alkoholysiert.